(19) Europäisches Patentamt
European Patent Office
Office européen des brevets

(11) Veröffentlichungsnummer: **0 128 459**
**A2**

(12) # EUROPÄISCHE PATENTANMELDUNG

(21) Anmeldenummer: **84106152.6**

(22) Anmeldetag: **30.05.84**

(51) Int. Cl.³: **A 61 K 31/415**, A 61 K 47/00

(30) Priorität: **10.06.83 DE 3321043**

(43) Veröffentlichungstag der Anmeldung: **19.12.84 Patentblatt 84/51**

(84) Benannte Vertragsstaaten: **AT BE CH DE FR GB IT LI LU NL SE**

(71) Anmelder: **BAYER AG, Konzernverwaltung RP Patentabteilung, D-5090 Leverkusen 1 Bayerwerk (DE)**

(72) Erfinder: **von Bittera, Miklos, Max-Scheler-Strasse 7, D-5090 Leverkusen 3 (DE)**
Erfinder: **Büchel, Karl-Heinz, Prof. Dr., Dabringhausener Strasse 42, D-5093 Burscheid (DE)**
Erfinder: **Plempel, Manfred, Dr., Zwengenbergstrasse 3c, D-5657 Haan (DE)**
Erfinder: **Regel, Erik, Dipl.-Ing., Untere Bergerheide 26, D-5600 Wuppertal 1 (DE)**

(54) **Antimykotische Mittel für einmalige gynäkologische Behandlung.**

(57) Die Erfindung betrifft antimykotische Mittel für die gynäkologische Anwendung durch einmalige Applikation mit höherer Bioverfügbarkeit der Wirkstoffe, enthaltend 1–10% Azolderivate und übliche Formulierungshilfsstoffe, und/oder eine Säure und/oder ein Puffersystem, bestehend aus einer organischen Säure und/oder deren Salzen, dadurch gekennzeichnet, daß sie den Wirkstoff in Partikeln 5 mµ ± 10% enthalten.

EP 0 128 459 A2

0128459

BAYER AKTIENGESELLSCHAFT 5090 Leverkusen, Bayerwerk
Konzernverwaltung RP
Patentabteilung Si/by-c

Antimykotische Mittel für einmalige gynäkologische Behandlung

Die vorliegende Erfindung betrifft neuartige Formulierungen der bekannten antimykotischen Azolderivate, die eine höhere Bioverfügbarkeit der Wirkstoffe aufweisen und dadurch eine gynäkologische Behandlung, die nur einmal angewendet werden muß, ermöglicht.

Für die Behandlung von Vaginal-Infektionen mit Pilzen sind bereits zahlreiche Zubereitungen von antimykotischen Azolderivaten bekannt geworden. Mit diesen Zubereitungen werden für eine vollständige Vaginal-Sanierung 14 bis 3 Tage Therapiezeit benötigt. Dies ist u.a. darauf zurückzuführen, daß in den üblichen Vaginalzubereitungen die enthaltenen Wirkstoffe nur zum Teil in wäßrigen Medien löslich sind.

Um zu einer Verkürzung der Therapiedauer zu kommen, benötigt man, besonders zur Eliminierung der Keime in Vaginalsekret, eine gewisse Depot-Wirkung und eine

0128459

höhere Bioverfügbarkeit der Wirkstoffe. Dafür sind die bekannten Formulierungen nur begrenzt geeignet, weil sich von dem vorhandenen Wirkstoffangebot nur ein kleiner Anteil im Flüssigkeitsvolumen der Vagina löst. Wenn man nun durch weitere Erhöhung der Wirkstoffkonzentration eine Verkürzung der Therapiedauer, z.B. auf einen Tag bei einmaliger Applikation, erreichen will, muß man für eine optimale Bioverfügbarkeit des Wirkstoffes Sorge tragen.

Es wurde nun gefunden, daß solche Creme- Schaum- bzw. Spray-Formulierungen antimykotischer Azolderivate, die den Wirkstoff in feinstgemahlener Form, vorzugsweise in Partikelgrößen mit einem mittleren Partikeldurchmesser von 5 m$\mu$ $\pm$ 10 %, die üblichen Formulierungshilfsstoffe und/oder eine Säure und/oder ein Puffersystem, bestehend aus einer organischen Säure und/oder deren Salzen enthalten, eine optimale Freisetzung des Wirkstoffes zeigen und damit eine auf einmalige Anwendung verkürzte Therapiedauer ermöglichen.

Wirkstoffe, die in dieser Weise formuliert werden können, sind alle antimykotisch wirksamen Derivate, insbesondere Imidazol- und Triazolderivate. Sie sind in den erfindungsgemäßen Mitteln in Mengen von 1 % bis 10 %, vorzugsweise von 5 bis 10 % vorhanden.

Beispielsweise seien die Verbindungen der nachstehenden Formeln genannt:

I 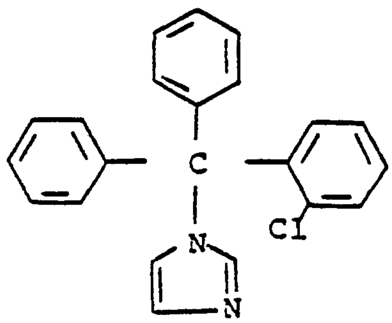 Clotrimazol

II 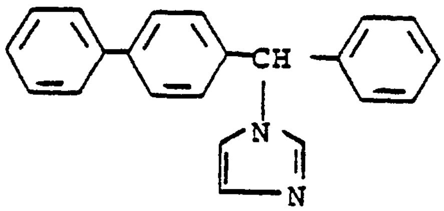 Bifonazol

III 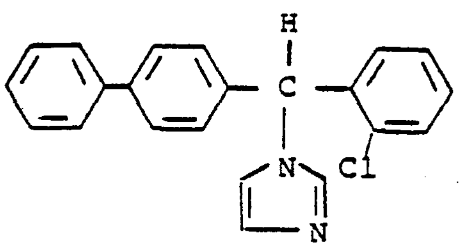 Lombazol

Zahlreiche weitere antimykotisch wirksame Azolderivate sind bekannt aus der DE-OS 24 30 039. Sie können ebenfalls in den erfindungsgemäßen Mitteln als Wirkstoffe dienen.

Insbesondere wurde gefunden, daß solche Vaginal-Creme- bzw. Sprayzubereitungen, die den Wirkstoff in feinstgemahlener Form, d.h. in Partikelgrößen 5 mμ ± 10 % oder die Verbindung der Formel II, die außerdem Milchsäure und Calciumacetat, oder der Formeln I und III, die außerdem primäres oder tertiäres Natriumcitrat enthalten, den Wirkstoff so optimal freisetzen, daß durch eine einmalige Applikation Vaginalmykosen, z.B. hervorgerufen durch Candida-Arten und Torulopsis-Arten, zur Abheilung gebracht werden können. Die Verträglichkeit solcher Formulierungen ist einwandfrei.

Auch andere Puffersysteme und/oder die Säuren oder die sauren Salze allein beeinflussen die Zubereitungen in der angegebenen günstigen Weise. Solche können sein:

| | | |
|---|---|---|
| Citronensäure | / | Na-citrat primär / Na-citrat tertiär |
| Milchsäure | / | Na-lactat |
| Weinsäure DL | / | K-Na-tartrat |
| Adipinsäure | | |
| Ascorbinsäure | / | Ethylendiamintetraessigsäure - 1/2 Na-salz bzw. Na-salz |
| Fumarsäure | | |
| Glykokoll-Puffer | | |
| Kaliumhydrogenphthalat-Puffer | | |
| Tartrat-Puffer ($KHC_2H_4O_6$) | | |
| Phosphat-Puffer | | |

Unter den üblichen Formulierungshilfsstoffen werden hier die nachstehenden verstanden.

Zunächst eignen sich Spreitmittel bzw. -öle.

Silikonöle verschiedener Viskosität.

Fettsäureester, wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, wachsartige Fettsäureester wie künstliches Entenbürzeldrüsenfett, Dibutylphthalat, Adipinsäurediisopropylester, letzterem verwandte Estergemische u.a..

Triglyceride, wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monoglyceride der $C_8$-$C_{10}$-Fettsäuren u.a.

Fettalkohole, wie Isotridecylalkohol, Cetylstearyl-Alkohol, Oleylalkohol.

Fettsäuren, wie z.B. Ölsäure.

Besonders gut geeignete spreitende Öle sind die folgenden: Isopropylmyristat, Isopropylpalmitat, Capryl/Caprinsäureester von gesättigten Fettalkoholen der

Kettenlänge $C_{12}$-$C_{18}$, wachsartige Fettsäureester wie künstliches Entenbürzeldrüsenfett, Silikonöle, Isopropylmyristat/Isopropylstearat/Isopropylpalmitat-Gemisch.

Glycerin, Paraffin dickflüssig, Paraffin dünnflüssig.

Für die erfindungsgemäßen Mittel eignen sich folgende Mittel als Emulgatoren:

Polyoxyethylen-Sorbitan-Fettsäureester, Kolloiddisperses Gemisch aus Cetyl-Stearylalkohol und Natrium-Cetyl-Stearylsulfat, Polyethylenstearat, Cetylstearylalkohol mit ca. 12 Mol Ethylenoxid, Cetylstearylalkohol mit ca. 30 Mol Ethylenoxid, Fettalkohol $C_{16}$-$C_{18}$ mit 25 Mol Ethylenoxid verethert, Sorbitan und Glycerinfettsäureester, ethoxyliertes Rizinusöl, Cetylstearylalkohol mit nicht ionogenem Emulgator-Zusatz.

Folgende weitere Hilfs- und/oder Formulierungs-Grundhilfsstoffe können bei der Herstellung der erfindungsgemäßen Mittel verwendet werden:

Tenside (beinhaltet Emulgatoren und Netzmittel), z.B.

1. anionaktive, wie Na-Laurylsulfat, Fettalkoholethersulfate, Mono/Dialkylpolyglykoletherorthophosphorsäureester-Monoethanolaminsalz;

2. kationaktive, wie Cetyltrimethylammoniumchlorid;

3. ampholytische, wie Di-N-Na-N-lauryl-ß-iminodipropionat oder Lecithin;

Beispiel 1

| | |
|---|---|
| Clotrimazol-Wirkstoff mikronisiert 5 mμ ± 10 % | 10,00 g |
| Sorbitan-Fettsäureester | 2,00 g |
| Polyoxyethylen-Sorbitan-Fettsäureester | 1,50 g |
| Walrat künstlich | 3,00 g |
| Cetylstearylalkohol | 3,50 g |
| Isopropylmyristat | 13,50 g |
| Benzylalkohol | 1,00 g |
| Wasser entmineralisiert | 65,50 g |

Phase I.

Sorbitan-Fettsäureester, Teilmenge Polyoxyethylen-Sorbitan-Fettsäureester, Walrat künstlich, Cetylstearylalkohol, Isopropylmyristat schmelzen, rühren, mischen bis 70°C.

Phase II.

Benzylalkohol mit Wasser mischen, aufkochen, abkühlen auf 75°C.

Phase II in Phase I langsam einrühren, auf 25°C abkühlen. Phase III, d.h. die Restmenge Polyoxyethylen-Sorbitan-Fettsäureester, in Wasser bei 50°C lösen, auf 25°C abkühlen, Clotrimazol-Wirkstoff mikronisiert zugeben, rühren, suspendieren und zu Phase I + II geben.

In analoger Weise wird bei den Beispielen 2 bis 5 gearbeitet.

Beispiel 2

| | |
|---|---|
| Bifonazol-Wirkstoff mikronisiert 5 mμ ± 10 % | 10,00 g |
| Sorbitan-Fettsäureester | 2,00 g |
| Polyoxyethylen-Sorbitan-Fettsäureester | 1,50 g |
| Walrat künstlich | 3,00 g |
| Cetylstearylalkohol | 3,50 g |
| Isopropylmyristat | 13,50 g |
| Milchsäure | 1,00 g |
| Ca-Lactat | 0,50 g |
| Benzylalkohol | 1,00 g |
| Wasser entmineralisiert | 64,00 g |

Beispiel 3

| | |
|---|---|
| Lombazol-Wirkstoff mikronisiert 5 mμ ± 10 % | 10,00 g |
| Sorbitan-Fettsäureester | 2,00 g |
| Polyoxyethylen-Sorbitan-Fettsäureester | 1,50 g |
| Walrat künstlich | 3,00 g |
| Cetylstearylalkohol | 6,00 g |
| Isopropylmyristat | 13,50 g |
| Milchsäure | 1,00 g |
| Na-Citrat tertiär | 1,00 g |
| Benzylalkohol | 1,00 g |
| Wasser entmineralisiert | 61,00 g |

Beispiel 4

| | |
|---|---|
| Clotrimazol-Wirkstoff mikronisiert 5 mµ ± 10 % | 10,00 g |
| Sorbitan-Fettsäureester | 2,00 g |
| Polyoxyethylen-Sorbitan-Fettsäureester | 1,50 g |
| Walrat künstlich | 3,00 g |
| Cetylstearylalkohol | 3,50 g |
| Isopropylmyristat | 13,50 g |
| Benzylalkohol | 1,00 g |
| Na-Citrat primär | 0,03 g |
| Wasser entmineralisiert | 65,47 g |

Beispiel 5

| | |
|---|---|
| Bifonazol-Wirkstoff mikronisiert 5 mµ ± 10 % | 5,00 g |
| Sorbitan-Fettsäureester | 2,00 g |
| Polyoxy-Sorbitan-Fettsäureester | 1,50 g |
| Walrat künstlich | 3,00 g |
| Cetylstearylalkohol | 6,00 g |
| Isopropylmyristat | 13,50 g |
| Benzylalkohol | 1,00 g |
| Milchsäure | 0,50 g |
| Calciumlactat | 0,25 g |
| Wasser entmineralisiert | 67,25 g |

Die so formulierten Zubereitungen werden in Applikatoren abgefüllt.

Beispiel 6

| | | |
|---|---|---|
| Teil A | Clotrimazol-Wirkstoff mikronisiert | |
| | 5 mμ ± 10 % | 10,000 g |
| | Glycerin wasserfrei | 1,000 g |
| | Na-Citrat primär | 0,025 g |
| | Wasser entmineralisiert | 84,775 g |
| | Solbrol M | 0,150 g |
| Teil B | Cetylstearylalkohol, mit nicht ionogenem Emulgator-Zusatz | 2,000 g |
| | Natrium-Cetylstearylsulfat | 1,000 g |
| | Ölsäuredecylester | 1,000 g |
| | Solbrol P | 0,050 g |

Teil B auf 60°C erwärmen, rühren. Bei Teil A Solbrol M bei 90°C lösen, auf 60°C abkühlen, Glycerin und Na-Citrat zugeben. Teil A in Teil B unter Vakuum einrühren, bei 40°C Clotrimazol zugeben, suspendieren auf 25°C abkühlen. Abfüllung in Aluminium-Dosen: 92 Teile Emulsion 8 Teile Treibgas 12/114 (40:60).
Die so hergestellten Sprays werden in "upside down" Stellung appliziert. Schaumvolumen ca. 40-45 ml.

Wirksamkeitstestung der erfindungsgemäßen Mittel am Trichophyton infizierten Meerschweinchen.

Als Testmodell zur vergleichenden Wirksamkeitsprüfung der erfindungsgemäßen Formulierungen verwendeten wir Trichophyton-infizierte Pirbright-white-Meerschweinchen

mit einem durchschnittlichen Gewicht von 600 g. Die Tiere wurden auf dem Rücken mit einer elektrischen Haarschneidemaschine so geschoren, daß ca. 1/10 mm lange Haarstümpfe stehen blieben. Die Infektion mit Trichophyton mentagrophytes erfolgte durch leichtes Verreiben einer 24 Stunden in Sabouraud-Nährlösung angekeimten Sporensuspension des Erregers auf einer ca. 2 x 2 cm großen Fläche des geschorenen Rückens der Tiere. Aufgetragen wurden pro Tier 0,5 ml Keimsuspension, die 1 - 3 x $10^5$ infektiöse Pilzpartikel enthielten.

Bei diesem Infektionsmodus zeigen sich 2-3 Tage post infectionem die ersten Symptome der Dermatophytose als Rötung und Schuppung der Haut. Bei unbehandelten Tieren ist ca. 14 Tage p.i. die Dermatophytose maximal ausgeprägt: flächiger Haarausfall und blutige Integument-Defekte innerhalb einer entzündlich veränderten, schuppigen Randzone.

Die zu prüfenden Formulierungen wurden 1-mal, am 2. Tag post infectionem, lokal auf die gerötete Infektionsstelle der Tiere appliziert und mit einem Hornspatel leicht verrieben.

Es wurden jeweils 0,5 g der Formulierungen = 50 mg Wirkstoff aufgetragen. Die Bewertung des Infektionsablaufs erfolgte täglich bis zum 20. Tag p.i.

Die Testergebnisse sind aus der nachstehenden Tabelle ersichtlich.

| Mittel des Beispiels | Wirkung am Trichophyton-infizierten Meerschweinchen |
|---|---|
| 1 | xxxx |
| 2 | xxxx |
| 3 | xxxx |
| 4 | xxxx |
| 5 | xxxx |
| 6 | xxxx |

xxxx: sehr gute Wirkung
xxx: gute Wirkung
xx: Wirkung
x: schwache Wirkung
O: keine Wirkung

Patentansprüche:

1. Antimykotische Mittel mit höherer Bioverfügbarkeit der Wirkstoffe, enthaltend 1 bis 10 % Azolderivate und übliche Formulierungshilfsstoffe, und/oder eine Säure und/oder ein Puffersystem, bestehend aus einer organischen Säure und/oder deren Salzen, dadurch gekennzeichnet, daß sie den Wirkstoff in Partikeln 5 mμ ± 10 % enthalten.

2. Antimykotische Mittel nach Anspruch 1, dadurch gekennzeichnet, daß sie den Wirkstoff in Partikeln 5 mμ ± 10 % und außerdem eine Säure und/oder ein Puffersystem, bestehend aus einer organischen Säure und/oder deren Salzen enthalten.

3. Antimykotische Mittel nach Anspruch 1, dadurch gekennzeichnet, daß sie als Wirkstoff Clotrimazol der Formel

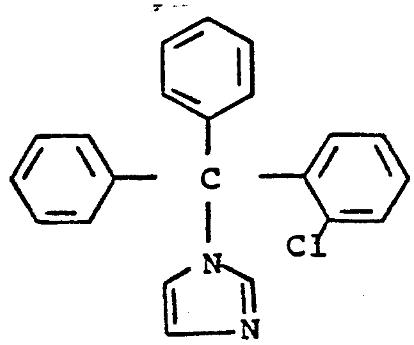

enthalten.

4. Antimykotische Mittel nach Anspruch 1, dadurch gekennzeichnet, daß sie als Wirkstoff Bifonazol der Formel

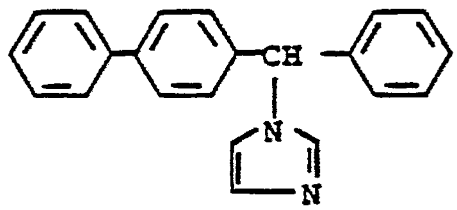

enthalten.

5. Antimykotische Mittel nach Anspruch 1, dadurch gekennzeichnet, daß sie als Wirkstoff Lombazol der Formel

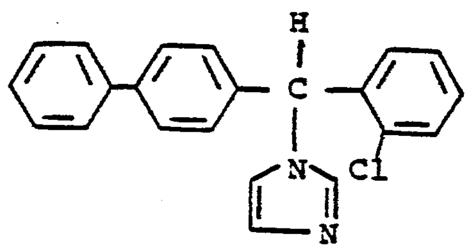

enthalten.

6. Antimykotische Mittel nach Anspruch 1, dadurch gekennzeichnet, daß es eine Creme ist.

7. Antimykotische Mittel nach Anspruch 1, dadurch gekennzeichnet, daß es ein Spray ist.

8. Antimykotische Mittel nach Anspruch 1, dadurch gekennzeichnet, daß sie den Wirkstoff in suspendierter Form enthalten.

9. Verfahren zur Herstellung von antimykotischen Mitteln nach Anspruch 1, dadurch gekennzeichnet, daß man antimykotische Azolderivate in an sich bekannter Weise synthetisiert, diese Azolderivate auf Teilchengrößen von 5 m/u ± 10 % zerkleinert und 1 bis 10 % der so zerkleinerten Azolderivate bei Temperaturen zwischen Zimmertemperatur und 70 °C in Gemische aus üblichen Formulierungshilfsstoffen und/oder einer Säure und/oder einem Puffersystem, bestehend aus einer organischen Säure und/oder deren Salzen, einarbeitet.

10. Verwendung von antimykotischen Mitteln nach Anspruch 1 bei der gynäkologischen einmaligen Applikation.